# EUROPEAN PATENT SPECIFICATION

(11) **EP 2 816 644 B1**
(45) Date of publication and mention of the grant of the patent: **16.08.2017**
(21) Application number: 13173170.5
(22) Date of filing: 21.06.2013
(51) Int. Cl.: H01M 4/38, H01M 4/60, H01M 10/052, C07C 255/31, C07C 255/61, H01M 10/0525

(54) **BATTERY COMPRISING A RADIALENE COMPOUND AS ELECTROACTIVE MATERIAL**
BATTERIE UMFASSEND EINE RADIALEN-VERBINDUNG ALS ELEKTROAKTIVES MATERIAL
BATTERIE COMPRENANT UN COMPOSÉ RADIALÈNE COMME MATÉRIAU ÉLECTROACTIF

(43) Date of publication of application: 24.12.2014
(73) Proprietor: Novaled GmbH, 01307 Dresden (DE)
(72) Inventor: Bouazza, Sofiane, 70771 Leinfelden (DE); Bobbio, Carla, 81543 München (DE); Werner, Ansgar, 01277 Dresden (DE)
(74) Representative: Bittner, Thomas L.

(56) References cited:
- EP-A1- 1 988 587
- JP-A- 2009 196 925
- JP-A- 2011 165 567
- JP-A- 2013 020 741
- US-A- 3 963 769
- US-A1- 2012 308 890
- V.R. KOCH ET AL: "NEW ANIONS AS SUPPORTING ELECTROLYTES FOR RECHARGEABLE LITHIUM BATTERIES", JOURNAL OF POWER SOURCES, vol. 20, no. 3/04, 1 January 1987 (1987-01-01), pages 287-291, XP001074282, ISSN: 0378-7753, DOI: 10.1016/0378-7753(87)80125-8
- V.E. DE OLIVEIRA ET AL: "Bis(dicyanomethylene)squarate squaraines in their 1,2- and 1,3-forms: Synthesis, crystal structure and spectroscopic study of compounds containing alkali metals and tetrabutylammonium ions", JOURNAL OF MOLECULAR STRUCTURE, vol. 936, no. 1-3, 7 August 2009 (2009-08-07), pages 239-249, XP026626241, ISSN: 0022-2860, DOI: 10.1016/J.MOLSTRUC.2009.08.002 [retrieved on 2009-08-07]
- O. CORTADELLAS ET AL: "Nickel coordination compounds of the cyanamido squarate ligand. The crystal structures of the 2,4-bis(cyanamido)cyclobutane-1,3-dione dianion (2,4-NCNsq<2->) and of three polymers with general formula [Ni(Him)x(H2O)4-x(2,4-NCNsq).yH2O]n (x=2-4; y=1-3; Him=imidazole)", INORGANICA CHIMICA ACTA, vol. 359, no. 2, 15 December 2005 (2005-12-15), pages 484-496, XP028068997, ISSN: 0020-1693, DOI: 10.1016/J.ICA.2005.07.048 [retrieved on 2006-01-20]
- I. DESPOTOVIC ET AL: "The structure and stability of [3]-radialenes and their dianions - A DFT study", JOURNAL OF MOLECULAR STRUCTURE (THEOCHEM), vol. 811, no. 1-3, 3 March 2007 (2007-03-03), pages 313-322, XP022070531, ISSN: 0166-1280, DOI: 10.1016/J.THEOCHEM.2007.01.045

## Description

### Technical field

The present invention relates to a battery comprising at least one electrochemical cell comprising a first electrode, a second electrode, a first electroactive material, a second electroactive material and an electrolyte which is in contact with both electrodes. The present invention also relates to electroactive materials as such and compounds suitable therefor.

### Background of the invention

Batteries comprising electrochemical cells serve widespread as mobile low-voltage direct current electricity sources. Present-day most frequently used secondary (rechargeable) batteries are lithium ion batteries. Such kind of battery can be built e.g. using transition metal oxide that contains lithium cations in the first electrode and crystalline or amorphous carbon in the second electrode as active materials, with a lithium salt as an electrolyte. The charging is then performed via connecting the first electrode to the positive pole of an external voltage source and the second electrode to the negative pole of the external voltage source, what results in an oxidation of the transition metal in the first electrode polarized as anode, deintercalation of the Li cations from the anode, their electromigration through the electrolyte, their reduction on the second electrode polarized as cathode, and in the intercalation of the formed electrically neutral Li atoms in the carbon matrix of the second electrode. When discharging this battery by connecting its poles to an external electrical resistance, the first electrode as the positive pole of the battery consumes electrons coming through the external circuit from the second electrode that represents the negative pole of the battery. The first electrode in the discharging battery is called cathode, because there takes place an electrochemical reduction of the transition metal to its lower valence, accompanied by intercalation of the Li cations form the electrolyte. The second electrode in the discharging battery is assigned, according to the electrochemical convention, as anode, because there takes place an electrochemical oxidation of the intercalated Li atoms, releasing electrons in the external circuit and Li cations in the electrolyte.

In batteries for the most of consumer devices, energy storage and operating time are the keys, so the more the better. Especially for mobile applications like cell phones, electric vehicles (EV) or hybrid vehicles (HEV), higher power and energy densities are required.

In this regard, electrochemically active materials play an essential role. For the use in rechargeable batteries, only highly reversible redox systems are applicable. For allowing high energy density, low molecular weight compounds exchanging high number of electrons per molecule or polymers with low molecular weight building units exchanging high number of electrons per building units are preferable, most preferably those showing, moreover, high redox potential. Among further (often somewhat contradictory) requirements on electroactive materials for batteries can be named their safety, intrinsic chemical stability and low aggressiveness, good mechanical properties, easy processability, environmental tolerability, abundance of necessary raw materials and low manufacturing cost. Solid or gel-like electroactive materials are preferred.

As an alternative to transition metal based inorganic redox-active materials, electroactive organic materials offer potential advantages in their light weight, low-temperature processability, raw material accessibility and lowering the environmental burden by absence of toxic metals like nickel. Examples of organic electroactive materials recommended for use in batteries are nitroxide compounds reported e.g. in US2004/115529 or tetracyanoquinodimethane (TCNQ) reported in Japanese patent application S57-210567. Yet, the performance of organic electroactive materials, especially in terms of their electrical properties, energy density, reversibility and stability, has to be significantly improved.

US 2012/308890 A1 discloses a lithium ion rechargeable battery comprising a cathode active material, a cathode for a lithium ion rechargeable battery using the cathode active material, the cathode active material including particles including a material capable of intercalating and deintercalating lithium ions and a film formed on at least one part of surfaces of the particles.

JP 2011-165567 A is related to an electrode active material, a power storage device, an electronic apparatus, and a transport apparatus. The electrode active material consists of an organic compound formed by a plurality of 1,3-dithiol ring molecules binding with a carbon atom of cycloalkane having a ring structure and the dithiol ring molecules being arranged in an annular form.

JP 2013-020741 A discloses a charge storage material, an electrode active material, an electrode and a battery. The charge storage material consists of a squaric acid derivative.

EP 1 988 587 A1 is related to oxocarbon, pseudo oxocarbon and radialene compounds and the use thereof.

JP 2009-196925 A discloses an organic salt useful as an ionic fluid and a method for producing said organic salt.

Koch et al., Journal of Power Sources, 20 (1987) 287-291 is related to new anions as supporting electrolytes for rechargeable lithium batteries. De Oliveira et al., Journal of Molecular Structure, 936 (2009) 239-249 discloses Bis(dicyanomethylene)squarate squaraines in their 1,2- and 1,3-forms. Further described in the synthesis, crystal structure and spectroscopic study of said compounds containing alkali metals and terbutylammonium ions.

Cortadellas et al., Inorganica Chimica Acta 359 (2006) 484-496 relates to nickel coordination compounds of the cyanamido squarate ligand. The crystal structures of exemplary compounds and three polymers comprising the same are shown.

Despotović et al., Journal of Molecular Structure: THEOCHEM 811 (2007) 313-322 is related to the structure and stability of [3]-radialenes and their dianions. Disclosed are DFT studies.

The object of the present invention is to provide batteries with improved energy density. Another object of the invention is to provide electroactive organic materials with improved energy density and sufficient stability and reversibility for their use in rechargeable batteries. Yet another object of the invention is to provide new compounds enabling the inventive electroactive organic material and batteries comprising it.

### Summary of the invention

This object is achieved by the subject-matter according to the independent claims. Preferred embodiments result from the sub-claims.

The object of the invention is achieved by battery comprising at least one electrochemical cell comprising a first electrode, a second electrode, a first electroactive material, a second electroactive material and an electrolyte which is in contact with both electrodes, wherein at least one of the first electroactive material and the second electroactive material comprises a radialene compound.

Preferably, the electroactive material comprising a radialene compound is the first electroactive material comprised in a first compartment of the electrochemical cell comprising the first electrode, and the first electrode is electrically connected with positive pole of the battery. In this embodiment, a reduction of an electroactive radialene compound runs on the first electrode, if the cell is operating in the discharging mode.

The radialene compound is selected from radialene compounds having their carbocyclic core substituted with groups independently selected from imino and methylidene, wherein the imino and methylidene groups are substituted with electron withdrawing substituents.

Preferably, the radialene compound is selected from [3]radialene and [4]radialene compounds having their cyclopropane or cyclobutane core substituted with groups independently selected from imino and methylidene, wherein the imino and methylidene groups are substituted with electron withdrawing substituents. Even more preferably, the electron withdrawing substituents are cyano groups.

Also preferably, the radialene compound has the general formula (I) 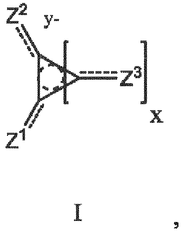 wherein Z¹, Z² and Z³ are independently selected from dicyanomethylidene and cyanimino group, x is an integer selected from 1 and 2, y is an integer selected from 0, 1 and 2 and the dashed lines represent
i) for y=0,
   6 electrons delocalized over 6 bonds if x = 1 and
   8 electrons delocalized over 8 bonds if x = 2,
ii) for y=1,
   7 electrons delocalized over 6 bonds if x = 1 and
   9 electrons delocalized over 8 bonds if x=2,
iii) for y = 2,
   8 electrons delocalized over 6 bonds if x = 1 and
   10 electrons delocalized over 8 bonds if x = 2.
More preferably, at least one group selected from Z¹ and Z² and at least one group Z³ is dicyanomethylidene.

Preferably, the electrolyte comprises alkali metal cations. Also preferably, the first electroactive material in its reduced state comprises alkali metal cations. Also preferably, the second electroactive material in its reduced state comprises an alkali metal. More preferably the alkali metal cation is Li⁺. Most preferably, the alkali metal is Li.

Another object of the invention is achieved by the electroactive material comprising an inventive radialene compound.

The electroactive material comprising a radialene compound is solid or gel-like.

Preferably, the radialene compound is selected from radialene compounds having their cycloalkane core substituted with groups independently selected from imino and methylidene, wherein the imino and methylidene groups are substituted with electron withdrawing substituents.

Also preferably, the radialene compound is selected from [3]radialene and [4]radialene compounds having their cyclopropane or cyclobutane core substituted with groups independently selected from imino and methylidene, wherein the imino and methylidene groups are substituted with electron withdrawing substituents. Even more preferably, the electron withdrawing substituents are cyano groups.

Even more preferably, at least one electroactive compound has the general formula (I) 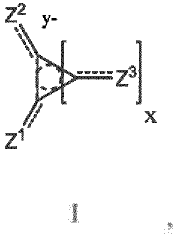 wherein Z¹, Z² and Z³ are independently selected from imino and methylidene, the imino and methylidene, groups are substituted with electron withdrawing substituents, x is an integer selected from 1 and 2, y is an integer selected from 0, 1 and 2 and the dashed lines represent
i) for y = 0,
   6 electrons delocalized over 6 bonds if x=1 and
   8 electrons delocalized over 8 bonds if x = 2,
ii) for y = 1,
   7 electrons delocalized over 6 bonds if x = 1 and
   electrons delocalized over 8 bonds if x = 2,
iii) for y = 2,
   8 electrons delocalized over 6 bonds if x = 1 and
   electrons delocalized over 8 bonds if x = 2.
Preferably, electron withdrawing groups are cyano groups. More preferably, at least one group selected from Z¹ and Z² and at least one group Z³ is dicyanomethylidene.

Also preferably, the electroactive material in its reduced state comprises alkali metal cations.

More preferably, the alkali metal cations are lithium cations.

Yet another object of the invention is achieved by the compound having formula (II), 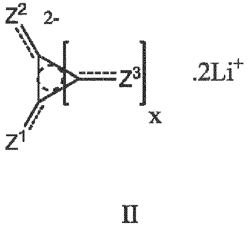 wherein Z¹, Z² and Z³ are independently selected from dicyanomethylidene, oxo and cyanimino group, x is an integer selected from 1 and 2 and the dashed lines represent
8 electrons delocalized over 6 bonds if x=1 and
10 electrons delocalized over 8 bonds if x=2, provided that in case that an oxo group is comprised in the compound one or two groups independently selected from Z¹, Z² and Z³ are oxo groups.

Preferably, at least one group selected from Z¹ and Z² and at least one group Z³ is dicyanomethylidene. Also preferably, one or two groups independently selected from Z¹, Z² and Z³ are oxo groups.

Examples of preferred compounds are

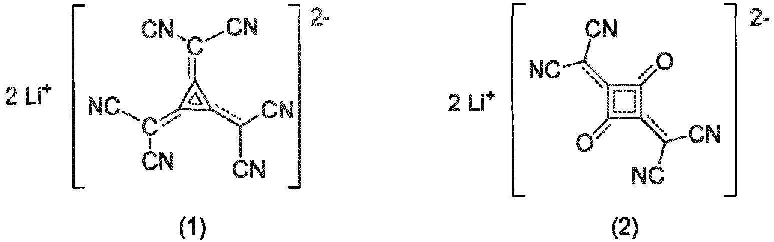

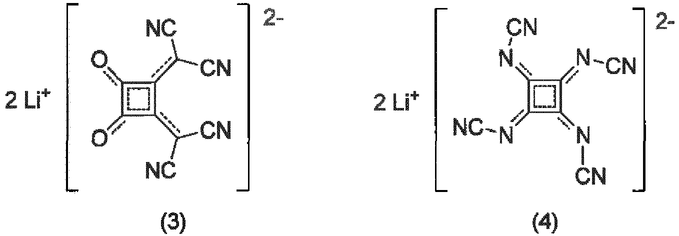

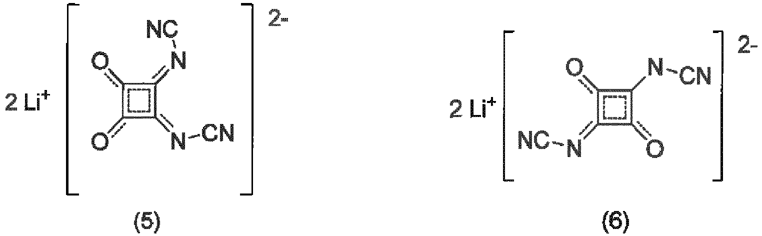

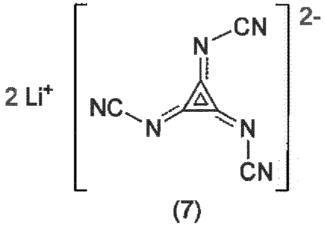

### Detailed description of the invention

Radialene compounds within this application are the compounds having a carbocyclic core in the shape of a convex regular polygon, wherein each core carbon atom is attached by sigma bonds to two of its neighbour core carbon atoms and to just one substituent placed outside the core and having its bond to the corner of the polygon in the radial direction from the centre of the polygon. To comply with the formal carbon valence which is equal four, the substituents are formally attached to the corners of the carbocyclic core by double bonds. Of course, pi-electrons in compounds formally described by means of alternating double and single bonds are more or less delocalized and a more accurate description of the bonding in them is possible e.g. in terms of resonance structures or molecular orbitals. The delocalization of pi-electrons in radialene compounds provides these compounds with specific properties. Fukunaga (J.Am.Chem.Soc. 1976, 98 (2), 610-611 and US 3 963 769) and Fukunaga et al (J.Am.Chem.Soc. 1976, 98 (2), 611-613 ) described tris(methylidene)cyclopropanes substituted with electron withdrawing groups as oxidants wherein the stronger electron withdrawing substituents are present, the more positive are the measured redox potentials. Such compounds could be potentially useful as electroactive component in electroactive materials for batteries.

Electroactive material is within this application defined as a condensed phase (solid or liquid) system comprising at least one electroactive component. Electroactive component is a compound which is able to create in an appropriately designed electrochemical cell a measurable voltage. In other words, all known oxidizing and/or reducing agents can be contemplated as potential electroactive compounds, however, compounds able to form reversible redox systems are strictly preferred. In rechargeable batteries, the best possible reversibility of electrochemical processes which take place in the used electroactive materials during battery charging and discharging is one of most important requirements for an industrially applicable electroactive material.

Unfortunately, all compounds reported by Fukunaga were not only very reactive, but also, with a sole exception of tris(diacetylmethylidene)cyclopropane which is a rather weak oxidant, intrinsically unstable. Especially, the most powerful oxidant tris(dicyanomethylidene)cyclopropane is a basically intractable compound that spontaneously decomposes under heating, reacts with common solvents and changes its colour quickly if stored in its solid state. Fukunaga reports its redox potential as high as + 1.13 V vs saturated calomel electrode (SCE) as reference what corresponds to a value about + 0.74 V vs ferroce-nium/ferrocene (Fc⁺/Fc) reference, nevertheless, he also reports that the measured cyclic voltammetry (CV) waves were not totally reversible.

Recently, the authors of the present application have confirmed that tris(dicyanomethylidene) cyclopropane is a very reactive and in its pure state synthetically difficult accessible strong oxidant. Nevertheless, they have surprisingly found that if the corresponding dianion occurs as an alkali metal salt in a solid or gel-like electroactive material that was placed in a cathode compartment of an electrochemical cell, the oxidized form created during the charging of the cell is more stable than a chemically prepared isolated compound, shows reversible redox electrochemical behavior and can be thus utilized as an electroactive component in rechargeable batteries. The same applies for various derivatives of general formula (I). It has been proven that through utilizing the inventive electroactive material in its electrochemically fully reduced state, the inventive electrochemical cell comprising compound (I) in the first electrode and/or in the first compartment adjacent to the first electrode can be safely built in its discharged state.

In electrochemical cells comprised in batteries, it is a widely used convention, to assign
- as a cathode the first electrode, wherein the spontaneous electrochemical reduction takes place during the discharging step, despite it is connected to the electrically positive electrical pole of the battery, and
- as an anode the second electrode, wherein the spontaneous electrochemical oxidation takes place during the discharging step, despite it is electrically connected to the electrically negative pole of the battery.

Thus, an appropriate first (cathode) electroactive material in the form prevailing in the charged cell shall be a strong oxidant, whereas in the reduced form prevailing in the discharged cell, it is a very weak reducing agent. Oppositely, an appropriate second (anode) electroactive material shall be in its reduced form prevailing in the charged cell a strong reducing agent, whereas in the oxidized form prevailing in the discharged cell, its oxidation ability is extremely weak.

The preferred inventive electroactive material perfectly fits with these requirements. In its fully reduced state, it comprises the compound (I) solely as a doubly charged anion having general formula (Ia), whereas in its electrochemically fully oxidized state, it comprises compound of the formula I solely in the electrically neutral form represented by general formula (Ib): 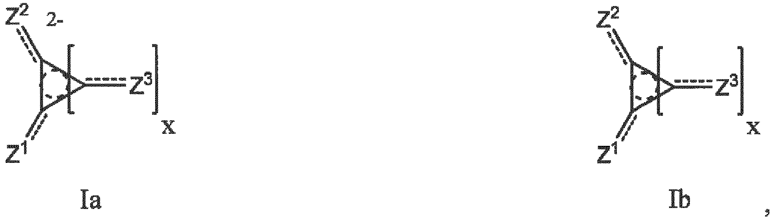 wherein Z¹, Z² and Z³ are independently selected from imino and methylidene, the imino and methylidene groups are substituted with electron withdrawing substituents, x is an integer selected from 1 and 2, and the dashed lines represent
i) for x=1,
   in compound (Ia) 8 electrons delocalized over 6 bonds, whereas in compound (Ib) only 6 electrons delocalized over 6 bonds,
ii) for x=2,
   in compound (Ia) 10 electrons delocalized over 8 bonds, whereas in compound (Ib) only 8 electrons delocalized over 8 bonds.

Electron withdrawing substituents are generally the substituents that posses positive value of sigma constant in the Hammet equation as taught in physical organic chemistry textbooks. Preferred are the substituents having the highest sigma values and the lowest relative molecular weights. Most preferred electron withdrawing group is the cyano group, therefore, the most preferred substituted imino and methylene groups are the cyanimino and dicyanomethylene groups.

Reduced form (Ia) is stable in form of salt with an appropriate cation. Preferred are salts with two alkali metal cations, more preferred are dilithium salts, even more preferred are the salts comprising two dicyanomethylene groups, as exemplified in formula (1a) or formulas (2a), (2a'):

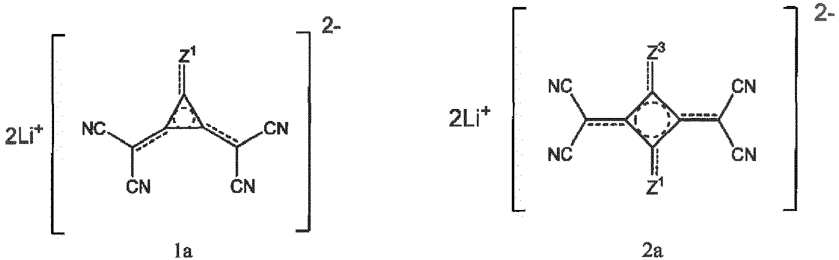

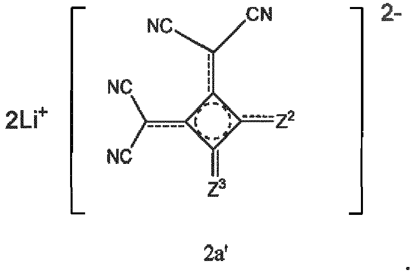

The oxidized electrically neutral form (Ib) can be for x = 1 shown in one of its mesomeric forms represented by formula (1b), for x = 2, in one of its mesomeric forms represented by formulas (2b) and (2c).

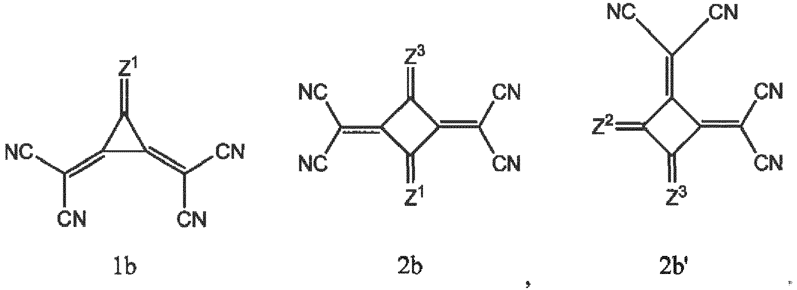

The voltammograms shown in the experimental part below bring an evidence that compound (I) with a single negative charge (y = 1) occurs as an intermediate form in the electrochemical reaction between the fully oxidized form (Ib) and fully reduced form (Ia). Nevertheless, it is supposed that it establishes the chemical equilibrium with both much more stable forms (Ia) and (Ib) with a rate high enough that it allows omitting the single-charged form from further consideration. The electrochemical potential of the inventive electroactive material according to the invention in any state between the fully oxidized state (Ib) and fully reduced state (Ia) is therefore given by the electrochemical equilibria of forms (Ia) and (Ib) in presence of other components of the inventive electroactive material.

Preferably, electroactive material comprising compound of formula (Ia) and/or (Ib) is comprised in the first electrode (cathode). Alternatively or additionaly, the electroactive material comprising compound of formula (Ia) and/or (Ib) may be comprised in the first (cathode) compartment of the electrochemical cell that is adjacent to the first electrode. It is preferred that the first compartment comprising compound of formula (Ia) and/or (Ib) is separated by a separator from the second compartment that is adjacent to the second electrode.

The inventive electroactive material may comprise electrochemically inactive components which do not change their redox state during operation of the inventive electrochemical cell. If the inventive electroactive material forms the first electrode, the electrochemically inactive components may be e.g. binders improving its mechanical properties or fillers improving its electrical properties. Typically, electrically conductive carbon black can be utilized as a filler improving conductivity, various polymers like polyethylene oxide, polyvinyl alcohol, polyacrylates, polyvinylidene fluoride, polyacrylonitrile and polysiloxanes may serve as binders. The electroactive material according to the invention may be also mixed with another electroactive material to prepare a composite first electrode.

Alternatively or in addition, the first electrode may comprise a metal, a semiconductor or another solid with good conductivity for electrons, which does not react with compound of formula (Ia) and/or (Ib) nor with other compounds comprised in the compartment adjacent to the first electrode. Preferably, the metal or the solid with metallic conductivity forms a continuous layer having direct electrical contact with the pole of the battery which belongs to the first electrode. Also preferably, the continuous layer with metallic conductivity consists of a conductive oxide, of carbon or of a chemically inert metal like platinum, gold or silver, or of a chemically inert metal alloy. In the combination with such inert electrode and with an appropriate separator, the compound (I) may also occur in the whole first compartment of the inventive electrochemical cell, partially or fully dissolved in the electrolyte, which in this case can be considered also as an electrochemically inactive component of the inventive electroactive material.

The electrolyte used in the battery cells of the present invention may be an electrolyte solution prepared by dissolving an electrolyte salt in an appropriate solvent. The example of particularly preferred solvent may be a room temperature ionic liquid (RTIL). Alternatively or in addition, low molecular non-ionic solvents may be used. Preferred are polar aprotic solvents like diethyl carbonate or ethylene carbonate. Polymers like polyethylene oxide or polyvinyl pyrrolidone may be comprised in the electrolyte as a solvent or a component of the solvent too.

As RTIL, preferred are imidazolium, pyrrolidinium, and aliphatic quaternary ammonium type ionic liquids comprising cations like 1-ethyl-3-methyl-imidazolium, 1-butyl-3-methylimidazolium, 1-butyl-2-methyl pyrrolidinium, N-methyl-N-propylpiperidinium, N,N-diethyl-N-methyl-N-(2-methoxyethyl) ammonium. The anionic component contained in the ionic liquid may be (CF₃SO₂)₂N⁻, (C₂F₆SO₂)₂N⁻, CF₃SO₃⁻, C(CF₃SO₂)₃⁻, PF₆⁻, BF₄⁻, and ClO₄⁻. These ionic liquids may be used alone or in combination with one or more other ionic liquids.

The electrolyte salt comprises the same cation that is chosen as the counter cation for the compound (Ia). Preferably, it is an alkali metal cation, more preferably, it is the lithium cation. Example of commercially available electrolyte salts comprising lithium cation are LiN(CF₃SO₂)₂, LiN(C₂F₅SO₂)₂, LiCF₃SO₃, LiC(CF₃SO₂)₃, LiPF₆, LiBF₄, and LiClO₄.

Alternatively (instead of the mentioned low molecular electrolyte salt) or in addition, solid polymeric electrolyte having the anionic groups covalently attached to the polymeric backbone may be used. It is preferred that the polymer used as the (co)solvent and/or as the electrolyte salt is crosslinked to form an infinite polymeric network.

A crosslinked and/or insoluble polymeric electrolyte may be used also as the separator dividing the electrochemical cell to two compartments - the first compartment adjacent to the first electrode and the second compartment adjacent to the second electrode. As a separator, an inert solid carrier like glas wool soaked with an appropriate liquid electrolyte like RTIL may be used as well.

The second electrode may be made of any commercially available materials currently supplied for use as anodes in discharging metal-ion batteries. Example of active materials for the second electrode include metal lithium, carbon materials, metal silicon, metal tin, metal aluminium, alloys of metals or semimetals like aluminum, lead, tin, silicon and magnesium with lithium, metal oxides like SnO₂, TiO₂, and conducting polymers like polyacetylene.

For better illustration of one of the preferred embodiments of the invention, the operation of a Li-ion rechargeable battery which comprises the electroactive compound of the formula (Ia) and/or (Ib) as an electroactive material in the first electrode and/or in the first compartment is explained below in detail.

During the charging of the inventive battery, the positive pole of the external voltage source is connected to the first electrode comprising a salt of formula (II). The salt is oxidized by supplying two electrons to the external circuit and releases its Li⁺ countercations into the surrounding electrolyte, affording the corresponding electrically neutral molecule (Ib). Alternatively, the salt (Ia) in the charged cell is dissolved in the first compartment, and when releasing two electrons through the chemically inert first electrode into the external circuit, it leaves the corresponding electrically neutral molecule (Ib) in the first compartment, either dissolved or precipitating as a solid. The released lithium cations migrate in both cases to the second electrode.

On the second electrode connected to the negative pole of the external voltage source, the cations from the electrolyte or cations incorporated in the body of the second electrode attach the electrons supplied from the external circuit and form an electrically neutral form of the second electroactive compound.

Oppositely, during discharging, the oxidized form (Ib) is reduced to corresponding dianion (Ia) that forms with two lithium cations the salt (II). The mechanism of the reversible energy storage in the inventive electrochemical cell is depicted in the Scheme 1.

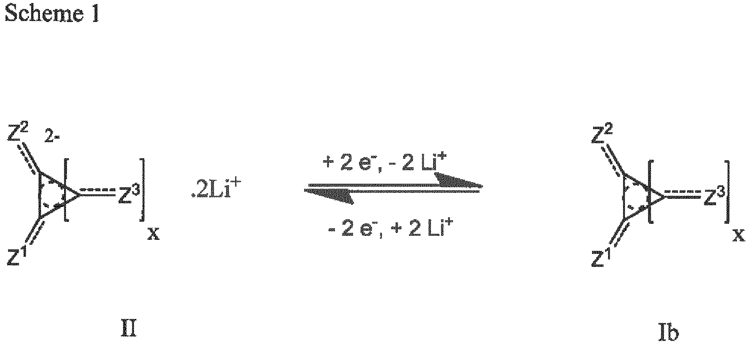

### Examples

### Example 1

### Dilithium salt of (3-(dicyanomethylene)cycloprop-1-ene-1,2-diyl)-bis-(dicyanomethanide) (1)

### Step 1, Tetrabutylammonium (TBA) salt of (3-(dicyanomethylene)cycloprop-1-ene-1,2-diyl)-bis-(dicyanomethanide) (Preparation is described in US3963769):

Under argon, sodium hydride (5.6 g, approx. 60 wt.% suspension in mineral oil, 7.0 eq) was washed with 3x30 mL hexanes, suspended in 130 mL anhydrous glyme and then cooled down to 4°C with an ice/water bath. In 15 min, redistilled malononitrile (4.22 g, 3.2 eq.) in 15 mL anhydrous glyme was added dropwise (gas evolution). The beige slurry was stirred 1 h at 0°C, before tetrachlorocyclopropene (2.45 mL, 1.0 eq) in anhydrous glyme (7.0 mL) is added slowly (during ca. 15 min, gas evolution) to the reaction mixture. The brown slurry was stirred 1 h at 0°C, then let warm up slowly to the room temperature (rt). Dropwise addition of 5 mL distilled water to quench and then diluting the reaction mixture with 70 mL distilled water was followed by addition of aqueous tetrabutylammonium bromide (TBABr, 14.1 g, 2.2 eq. previously dissolved in 15 mL distilled water). The resulting slurry was stirred for 2 h and the formed precipitate was filtered. After thoroughly washing with water (5x30 mL), the brown precipitate was dried in vacuo or at air overnight. The solid was then dissolved in 200 mL acetonitrile (AN) and re-precipitated by addition 600 mL ethyl acetate (EA). Precipitation can be repeated once more to get a colorless solid.
Yield: 12.1 g (85%)
Analytics:
ESI-MS (in 100% AN) neg: 470,228.
IR (solid, measured in the attenuated total reflectance (ATR) arrangement): 2962m, 2936m, 2865m, 2182s, 2163s, 1412s cm⁻¹.

### Step 2, Lithium salt of (3-(dicyanomethylene)cyclopmp-1-ene-1,2-diyl)bis-(dicyano-methanide), Li₂C₁₂N₆ (1):

Tetrabutylammonium (3-(dicyanomethylene)cycloprop-1-ene-1,2-diyl)bis(dicyano metha-nide) (12.0 g, 17.0 mmol) was dissolved in 230 mL AN, a solution 11.4 g lithium iodide (5.0 eq.) in 50 mL AN was added and the reaction mixture was heated at 80°C under inert atmosphere for 20 h. The reaction mixture was cooled down to approx. 50°C and the pale yellow precipitate was filtered under inert atmosphere. Washing with 3x15 mL AN and drying in vacuo afforded 2.65 g (64%) pale yellow solid.
Analytics:
ESI-MS (in 100% AN) neg: 228,114 m/z.
IR (solid, ATR): 2223s, 2195s, 2170s, 1638m, 1425s cm⁻¹

### Example 2

### Lithium salt of 2,2'-(2,4-dioxo-1,3-cyclobutanediyl)bis-propanedinitrile (2) Step 1, Sodium salt of 2,2'-(2,4-dioxo-1,3-cyclobutanediyl)bis-propanedinitrile:

0.94 g Sodium was dissolved in 100 mL dry ethanol and stirred over night. 3.1 g starting betaine 1,3-bis(dimethylamino)-2,4-dihydroxy-cyclo-butane-diylium (18.5 mmol) was added und the mixture stirred for 15 min, followed by addition of 2.44 g malonitrile. The mixture was refluxed for 3.5 h. After cooling to rt, the solid was filtered off and washed with ethanol. Drying was done in vacuum at 50°C/10 mbar over night.
Yield: 4.9 g (79%)
Analytics:
ESI-MS (in 100% AN) neg: 207.

### Step 2, Tetrabutyl ammonium salt of 2,2'-(2,4-dioxo-1,3-cyclobutanediyl)bis-propanedinitrile:

The sodium salt of 2,2'-(2,4-dioxo-1,3-cyclobutanediyl)bis-propanedinitrile (2.9 g) was suspended in 60 mL water and 7.3 g TBABr dissolved in 60 mL chloroform were added at rt and stirred for 2 h. The mixture was poured into a separator funnel and three phases were obtained. The solid was filtered off, washed with small portions water and chloroform, and dried on a clay plate, then in vacuum at 50°C/10 mbar over night to give 3.9 g yellow powder. A cca 1.4 second crop of the product was obtained after stirring the filtrate for 1 week, filtration, washing with small portions chloroform and drying in vacuum at 50°C/10 mbar over night.
Yield: 3.9 g (50%, the first crop only)
Analytics:
ESI-MS (in 100% AN) neg: 208, 450.
IR (solid, ATR): 2961m, 2874m, 2176s, 2155s, 1580s, 1369 cm⁻¹,

### Step 3, Lithium salt of 2,2'-(2,4-dioxo-1,3-cyclobutanediyl)bis-propanedinitrile (2):

In a glove box, TBA salt of 2,2'-(2,4-dioxo-1,3-cyclobutanediyl)bis-propanedinitrile (0.333 g/0.48 mmol) and LiI (0.300 g/2.24 mmol) were placed in a flask and 7.5 mL acetonitrile were added. A yellow precipitate occurred immediately. The mixture was allowed to stir over night. The precipitate was filtered off, washed with AN and dried in vacuum at 50°C/10 mbar over night to give 0.12 g (82%) yellow solid.
Analytics:
ESI-MS (100% AN) neg: 208.
IR (solid): 2196s, 1594m, 1543s, 1383s, 1364s cm⁻¹.

### Example 3 (CV measurement)

Cyclic voltammetry (CV) was performed using a three electrode glass cell using Potenti-ostat/Galvanostat PGSTAT 30. Pt disc d = 1 mm was used as working electrode, Pt wire as counter electrode and Ag/AgCl as reference electrode, and 0.1M LiPF₆ in ethylene carbonate: diethyl carbonate EC:DEC (v/v = 1/2) was used as electrolyte. The CV measurements were done at the scan rate 50 mV/s and rt.

### Example 4 (cathode electrode preparation for battery test)

The obtained materials of the present invention were eventually mixed with carbon and ethylene propylene diene monomer (EPDM) in a weight ratio 50:40:10 in cyclohexane to form a slurry. The obtained slurry was coated on aluminum foil using Doctor Blade and was dried to make the cathode electrode.

### Example 5 (charging/Discharging tests)

The charging/discharging tests were performed using a Swagelock-type cell assembled under argon. The testing cells were prepared by sandwiching the separator filled with electrolyte between lithium foil 1.2 mm and the prepared composite cathode electrode (Example 3). Glass fiber (GF/D) from Whatman® was used as a separator. Lithium foil was used as counter and reference electrodes. A solution of 1 M LiN(CF₃SO₂)₂ in 1-ethyl-3-methylimidazolium bis(trifluoromethylsulfonyl)imide was used as electrolyte. Charge/discharge cycling was carried out between 2.8 and 4.2 V using a BaSyTec cell test system (BaSyTec GmbH).

The cyclic voltammogram recorded for 2,2',2"-(cyclopropane-1,2,3-triylidene)trimalononitrile bis lithium salt (1) demonstrates reversible redox system featuring the transfer of two electrons, as indicated by two oxidation peaks at the anodic scan and two reduction peaks during cathode scan. From the CV data, it is clear that Li₂C₁₂N₆ (1) shows a fully reversible redox behaviour, an important condition for applicability of this compound as active material in a rechargeable battery.

From the first charge/discharge curves of (1) as cathode active material and lithium as anode at C/10 and C/2 rates respectively, at charging state one can see two plateaus at around 3.55 and 4 V corresponding to the oxidation of Li₂C₁₂N₆ under release of two lithium cations and formation of C₁₂N₆, at the discharging curve corresponding to the reduction of C₁₂N₆ to Li₂Cz₂N₆, two other plateaus are observed at 3.75 and 3.2 V.

The secondary battery cell using (1) as cathode material provides a high discharging capacity of 193 mAh/g at C/10 and 186 mAh/g at C/2.

It is clear from the electrochemical tests that the use of compound (1) in the cathode provides a rechargeable lithium ion battery with a higher charging and discharging voltage. The voltage of the electrochemical cell using Li₂C₁₂N₆ as cathode and lithium as anode after charging was 3.8V and no self discharge was observed.

### [4]radialene (2) (2,2'-(2,4-dioxocyclobutane-1,3-diylidene)dimalononitrile bis lithium salt) as cathode material (not according to the invention)

The first charge/discharge curves of the Li₂C₁₀N₄O₂ (2) as cathode active material and lithium as anode at C/10 and C/2 were measured. In both curves, during charging of the electrochemical cell, two plateaus are identified at around 3.6 and 4V corresponding to the oxidation of Li₂C₁₂N₆ (release of two lithium cations and formation of C₁₂N₆), at the discharging curve two other plateaus are observed at 3.87 and 3.2 V. The electrochemical cell using (2) as cathode material provides the discharging capacity 82 mAh/g at C/10 and 70 mAh/g at C/2.

The variation of discharge capacity with the number of cycles for the battery cell using (2) as cathode active material at C/10 was measured. A 67% retention of the initial capacity was observed after 12 cycles.

The features disclosed in the foregoing description, in the claims and in the accompanying drawings may, both separately or in any combination be material for realizing the invention in diverse forms thereof.

## Claims

1. Battery comprising at least one electrochemical cell comprising a first electrode, a second electrode, a first electroactive material, a second electroactive material and an electrolyte which is in contact with both electrodes, wherein at least one of the first electroactive material and the second electroactive material comprises a radialene compound, wherein the radialene compound is selected from radialene compounds having their carbocyclic core substituted with groups independently selected from imino and methylidene, wherein the imino and methylidene groups are substituted with electron withdrawing substituents.

2. Battery according to claim 1, wherein the electroactive material comprising a radialene compound is the first electroactive material comprised in a first compartment of the electrochemical cell comprising the first electrode, and the first electrode is electrically connected with the positive pole of the battery.

3. Battery according to claim 1, wherein the electron withdrawing substituents are cyano groups.

4. Battery according to any of claims 1 - 3, wherein the radialene compound has the general formula (I) 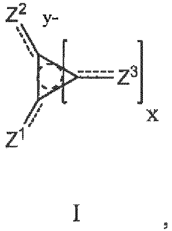 wherein Z¹, Z² and Z³ are independently selected from dicyanomethylidene and cyanimino group, x is an integer selected from 1 and 2, y is an integer selected from 0,1 and 2 and the dashed lines represent
i) for y = 0,
6 electrons delocalized over 6 bonds if x=1 and
8 electrons delocalized over 8 bonds if x=2,
ii) for y=1,
7 electrons delocalized over 6 bonds if x = 1 and
9 electrons delocalized over 8 bonds if x = 2,
iii) for y = 2,
8 electrons delocalized over 6 bonds if x=1 and
10 electrons delocalized over 8 bonds if x = 2.

5. Battery according to claim 4, wherein at least one group selected from Z¹ and Z² and at least one group Z³ is dicyanomethylidene.

6. Battery according to any of claims 1 - 5, wherein the electrolyte comprises alkali metal cations.

7. Battery according to any of claims 1-6, wherein the first electro active material in its reduced state comprises alkali metal cations.

8. Battery according to any of claims 1-5, wherein the second electroactive material in its reduced state comprises an alkali metal.

9. Battery according to claim 7, wherein alkali metal cations are lithium cations.

10. Battery according to claim 8, wherein the alkali metal is Li.

11. Electroactive material comprising a radialene compound as defined in any of the claims 1 to 10, wherein the electroactive material is solid or gel-like.

12. Compound having formula (II), 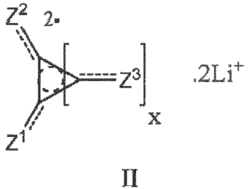 wherein Z¹ Z² and Z³ are independently selected from dicyanomethylidene, oxo and cyanimino group, x is an integer selected from 1 and 2 and the dashed lines represent 8 electrons delocalized over 6 bonds if x = 1 and
10 electrons delocalized over 8 bonds if x = 2, provided that in case that an oxo group is comprised in the compound one or two groups independently selected from Z¹, Z² and Z³ are oxo groups.

13. Compound according to claim 12, wherein at least one group selected from Z¹ and Z² and at least one group Z³ is dicyanomethylidene, and preferably one or two groups independently selected from Z¹, Z² and Z³ are oxo groups.

## Patentansprüche

1. Batterie, umfassend zumindest eine elektrochemische Zelle, umfassend eine erste Elektrode, eine zweite Elektrode, ein erstes elektroaktives Material, ein zweites elektroaktives Material und einen Elektrolyten, der in Kontakt mit beiden Elektroden steht, wobei zumindest eines des ersten elektroaktiven Materials und des zweiten elektroaktiven Materials eine Radialenverbindung umfasst, wobei die Radialenverbindung ausgewählt ist aus Radialenverbindungen, deren carbozyklischer Kern mit Gruppen substituiert ist, die ausgewählt sind aus Imino und Methyliden, wobei die Imino- und Methylidengruppen substituiert sind mit elektronenziehenden Substituenten.

2. Batterie nach Anspruch 1, wobei das elektroaktive Material, umfassend eine Radialenverbindung, ein erstes elektroaktives Material ist, das in einem ersten Abschnitt der elektrochemischen Zelle, umfassend die erste Elektrode, umfasst ist, und die erste Elektrode elektrisch mit den positiven Polen der Batterie verbunden ist.

3. Batterie nach Anspruch 1, wobei die elektronenziehenden Substituenten Cyanogruppen sind.

4. Batterie nach einem der Ansprüche 1 bis 3, wobei die Radialenverbindung die allgemeine Formel (I) hat 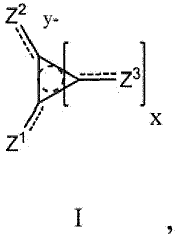 wobei Z¹, Z² und Z³ unabhängig ausgewählt sind aus Dicyanomethyliden und Cyaniminogruppen, x eine ganze Zahl, ausgewählt aus 1 und 2, ist, y eine ganze Zahl, ausgewählt aus 0, 1 und 2, ist und die gestrichelte Linie darstellt
i) für y = 0,
6 Elektronen, die über 6 Bindungen delokalisiert sind, wenn x = 1 und
8 Elektronen, die über 8 Bindungen delokalisiert sind, wenn x = 2,
ii) für y=1,
7 Elektronen, die über 6 Bindungen delokalisiert sind, wenn x = 1 und
9 Elektronen, die über 8 Bindungen delokalisiert sind, wenn x = 2,
iii) für y = 2
8 Elektronen, die über 6 Bindungen delokalisiert sind, wenn x = 1 und
10 Elektronen, die über 8 Bindungen delokalisiert sind, wenn x = 2.

5. Batterie nach Anspruch 4, wobei zumindest eine Gruppe, ausgewählt aus Z¹ und Z² und zumindest eine Gruppe Z³ Dicyanomethyliden ist.

6. Batterie nach einem der Ansprüche 1 bis 5, wobei der Elektrolyt Alkalimetallkationen umfasst.

7. Batterie nach einem der Ansprüche 1 bis 6, wobei das erste elektroaktive Material in seinem reduzierten Zustand Alkalimetallkationen umfasst.

8. Batterie nach einem der Ansprüche 1 bis 5, wobei das zweite elektroaktive Material in seinem reduzierten Zustand ein Alkalimetall umfasst.

9. Batterie nach Anspruch 7, wobei Alkalimetallkationen Lithiumkationen sind.

10. Batterie nach Anspruch 8, wobei das Alkalimetall Li ist.

11. Elektroaktives Material, umfassend eine Radialenverbindung wie in einem der Ansprüche 1 bis 10 definiert, wobei das elektroaktive Material ein Feststoff oder gelartig ist.

12. Verbindung der Formel (II), 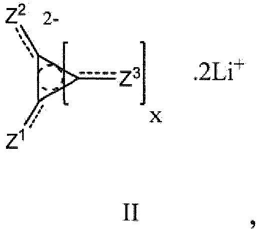 wobei Z¹, Z² und Z³ unabhängig ausgewählt sind aus Dicyanomethyliden, Oxo und Cyaniminogruppe, x zumindest eine ganze Zahl ist, die ausgewählt ist aus 1 und 2, und die gestrichelte Linie darstellt
8 Elektronen, die über 6 Bindungen delokalisiert sind, wenn x = 1 und
10 Elektronen, die über 8 Bindungen delokalisiert sind, wenn x = 2, vorausgesetzt, dass in dem Fall, dass eine Oxogruppe in der Verbindung umfasst ist, eine oder zwei Gruppen unabhängig ausgewählt aus Z¹, Z² und Z³ Oxogruppen sind.

13. Verbindung nach Anspruch 12, wobei zumindest eine Gruppe, ausgewählt aus Z¹ und Z², und zumindest eine Gruppe Z³ Dicyanomethyliden, und vorzugsweise eine oder zwei Gruppen, unabhängig ausgewählt aus Z¹, Z² und Z³, Oxogruppen sind.

## Revendications

1. Batterie comprenant au moins une cellule électrochimique comprenant une première électrode, une seconde électrode, un premier matériau électroactif, un second matériau électroactif et un électrolyte qui est en contact avec les deux électrodes, dans lequel au moins un du premier matériau électroactif et du second matériau électroactif comprend un composé radialène, dans lequel le composé radialène est sélectionné parmi les composés radialène ayant leur noyau carbocyclique substitué par des groupes indépendamment sélectionnés parmi l'imino et le métllylidéne, dans lequel les groupes imino et rnéthylidène sont substitué par des substituants attracteurs d'électrons.

2. Batterie selon la revendication 1, dans lequel le matériau électroactif comprenant un composé radialène est le premier matériau électroactif compris dans un premier compartiment de la cellule électroactif comprenant la première électrode, et la première électrode est connecte électriquement avec le pôle positif de la batterie.

3. Batterie selon la revendication 1, dans lequel les substituants attracteurs d'électrons sont des groupes cyano.

4. Batterie selon une quelconque des revendications 1-3, dans lequel le composé radialène a une formule générale (1) 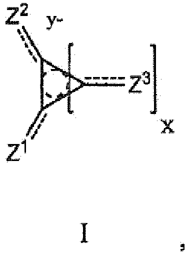 dans lequel Z¹, Z² et Z³ sont sélectionnés indépendamment du groupe dicyanométhylidène et cyanimino, x est un entier sélectionné parmi 1 et 2, y est un entier sélectionné parmi 0,1 et 2 et les lignes en pointillés représentent
i) pour y=0,
6 électrons délocalisés sur 6 liaisons si x=1 et
8 électrons délocalisés sur 8 liaisons si x=2,
ii) pour y=1,
7 électrons délocalisés sur 6 liaisons si x=1 et
9 électrons délocalisés sur 8 liaisons si x=2,
iii) pour y=2,
8 électrons délocalisés sur 6 liaisons si x=1 et
10 électrons délocalisés sur 8 liaisons si x=2.

5. Batterie selon la revendication 4, dans lequel au moins un groupe sélectionné parmi Z¹ et Z² et au moins un groupe Z³ est du dicyanométhylidène.

6. Batterie selon une quelconque des revendications 1-5, dans lequel l'électrolyte comprend des cations de métal alcalin.

7. Batterie selon une quelconque des revendications 1-6, dans lequel le premier matériau électroactif dans son état réduit comprend des cations de métal alcalin.

8. Batterie selon une quelconque des revendications 1-5, dans lequel le second matériau électroactif dabs son état réduit comprend un métal alcalin.

9. Batterie selon la revendication 7, dans lequel des cations de métal alcalin sont des cations de lithium.

10. Batterie selon la revendication 8, dans lequel le métal alcalin est Li.

11. Matériau électroactif comprenant un composé radialène tel que défini dans une quelconque des revendications 1 à 10, dans lequel le matériau électrolyte est solide ou de type gel.

12. Composé ayant une formule (II), 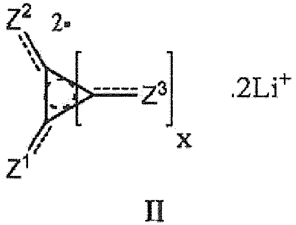 dans lequel Z¹, Z² et Z³ sont sélectionnés indépendamment parmi le groupe dicyanométhylidène, oxo et cyanimino, x est un entier sélectionné parmi 1 et 2 et les lignes en pointillés représentent
8 électrons délocalisés sur 6 liaisons si x=1 et
10 électrons délocalisés sur 8 liaisons si x=2, à condition que dans le cas où un groupe oxo est compris dans le composé, un ou deux groupes indépendamment sélectionnés parmi Z¹, Z² et Z³ et des groupes oxo.

13. Composé selon la revendication 12, dans lequel au moins un groupe sélectionné parmi Z¹ et Z² et au moins un groupe Z³ est du dicyanométhylidène, et de préférence un ou deux groupes sélectionnés indépendamment parmi des groupes Z¹, Z² et Z³ et oxo.
